# EUROPEAN PATENT APPLICATION

(11) **EP 1 788 084 A1**
(43) Date of publication of application: **23.05.2007**
(21) Application number: 05768643.8
(22) Date of filing: 02.08.2005
(51) Int. Cl.: C12N 15/09, C12N 15/81, C12N 1/19, C12P 21/02

(54) **YEAST PROMOTER**

(30) Priority: 06.08.2004 JP 2004230227
(71) Applicant: Juridical Foundation The Chemo-Sero-Therapeutic Research Institute, Kumamoto-shi Kumamoto-ken 860-8568 (JP)
(72) Inventor: Meta, Akihiro, The Chemo-Sero-Therapeutic Res. Inst., Kikuchi-shi, Kumamoto-ken 869-1298 (JP); Nakahara, Yo, Juridical Foundation The Chemo-Sero, Kikuchi-shi, Kumamoto-ken 869-1298 (JP); Higuchi, Hirofumi, The Chemo-Sero-Therapeutic Res. Inst., Kikuchi-shi, Kumamoto-ken 869-1298 (JP)
(74) Representative: Vossius & Partner
(86) International application number: PCT/JP2005/014119
(87) International publication number: WO 2006/013859

(57) **Abstract**

The present invention provides a DNA fragment exhibiting a potent promoter activity when used in the yeast. The present invention relates to a DNA fragment possessing a transcription promoter activity of the yeast, said DNA fragment having a whole of the DNA sequence shown by SEQ ID NO: 1 or a portion of said DNA sequence inclusive of its 3'-end, or having a DNA sequence which is hybridizable to a sequence complementary to a whole of the DNA sequence shown by SEQ ID NO: 1 or to a portion of said DNA sequence inclusive of its 3'-end and maintains the transcription promoter activity of said whole or portion of the DNA sequence shown by SEQ ID NO: 1; an expression plasmid containing a recombinant DNA fragment comprising said DNA fragment and a gene coding for a heterologous protein positioned downstream of said DNA sequence; a host cell transformed with said recombinant DNA fragment or said expression plasmid; and a method for preparing a heterologous protein which comprises culturing said host cell and recovering the heterologous protein from the culture. According to the present invention, a potent promoter usable in a heterologous gene expression system with the yeast as a host is provided.

## Description

### TECHNICAL FIELD

The present invention relates to a novel yeast promoter. More particularly, the present invention relates to a novel DNA sequence possessing a transcription promoter activity, an expression vector comprising said sequence, and its use for the production of a recombinant protein, e.g. a. heterologous protein. Also, the present invention relates to a recombinant cell comprising said DNA sequence.

### BACKGROUND ART

In recent years, through application of the genetic engineering technique to yeast, attempts have been done to prepare yeast strains that produce a large quantity of a useful protein such as medicaments or food. For the production of a heterologous protein, the technique is known to introduce a heterologous gene encoding said heterologous protein into the yeast Saccharomyces, inter alia Saccharomyces cerevisiae (hereinafter referred to as "S. cerevisiae"). For the expression of a heterologous gene, including a genomic DNA and a cDNA, in S. cerevisiae, a "promoter" needs be bound upstream said heterologous gene that allows for expression of said gene in the yeast.

A desired promoter is one that directs expression in the yeast of a protein of interest in a large quantity and several powerful promoters are known up till the present. For instance, a promoter of 3-phosphoglycerate kinase (PGK1) gene (Non-patent reference 1) and a promoter of a translation elongation factor (TEF1) (Non-patent reference 2), which are known to exhibit a high expression level when used as a promoter in S. cerevisiae, have been used for the expression of a heterologous gene in the yeast.
Non-patent reference 1: Ogden et al., 1986, Mo1 Cell Biol., 6(12), p. 4335-4343
Non-patent reference 2: Cottrelle et al., 1985, J. Biological Chemistry, 260(5), p. 3090-3096

### DISCLOSURE OF THE INVENTION

### (Technical Problem to be Solved by the Invention)

An object of the present invention is to provide a more potent promoter than the convention promoters usable in a heterologous gene expression system with yeast as a host.

### (Means for Solving the Problems)

In order to attain the object described above, the present inventors have carried out intensive investigation on the gene expression in S. cerevisiae and as a consequence succeeded in obtaining a DNA fragment comprising a potent promoter from the chromosome of S. cerevisiae to thereby complete the present invention.

Thus, the present invention relates to a DNA fragment possessing a transcription promoter activity of yeast, said DNA fragment having a whole of the DNA sequence shown by SEQ ID NO: 1 or a portion, of said DNA sequence inclusive of its 3'-end, or having a DNA sequence which is hybridizable to a sequence complementary to a whole of the DNA sequence shown by SEQ ID NO: 1 or a portion of said DNA sequence inclusive of its 3'-end and maintains the transcription promoter activity of said whole or portion of the DNA sequence shown by SEQ ID NO: 1. The present invention also relates to an expression plasmid comprising said DNA sequence and a heterologous gene positioned downstream of said DNA sequence. The present invention further relates to a transformant obtained by transformation of a host cell with said expression plasmid. The present invention still further relates to a method for preparing a heterologous protein which comprises culturing said transformant and recovering the heterologous protein from the culture.

Specifically, the present invention encompasses the following embodiments (A) to (K).
(A) A DNA fragment possessing a transcription promoter activity of yeast, said DNA fragment having a whole of the DNA sequence shown by SEQ ID NO: 1 or a portion of said DNA sequence inclusive of its 3'-end, or having a DNA sequence which is hybridizable to a sequence complementary to a whole of the DNA sequence shown by SEQ ID NO: 1 or to a portion of said DNA sequence inclusive of its 3'-end and maintains the transcription promoter activity of said whole or portion of the DNA sequence shown by SEQ ID NO: 1;
(B) The DNA fragment of (A) wherein said portion of the DNA sequence is a sequence comprising at least 650 bp from the 3'-end of the DNA sequence shown by SEQ ID NO: 1 (i.e. SEQ ID NO: 6; corresponding to the nucleotides of from No. 751 to No. 1400 in SEQ ID NO: 1) ;
(C) The DNA fragment of (A) or (B) wherein said portion of the DNA sequence is a sequence comprising at least 820 bp from the 3'-end of the DNA sequence shown by SEQ ID NO: 1 (i.e. SEQ ID NO: 5; corresponding to the nucleotides of from No. 581 to No. 1400 in SEQ ID NO: 1);
(D) The DNA fragment of any one of (A) to (C) wherein said portion of the DNA sequence is a sequence comprising at least 950 bp from the 3'-end of the DNA sequence shown by SEQ ID NO: 1 (i.e. SEQ ID NO: 4; corresponding to the nucleotides of from No. 451 to No. 1400 in SEQ ID NO: 1);
(E) The DNA fragment of any one of (A) to (D) wherein said portion of the DNA sequence is a sequence comprising at least 1100 bp from the 3'-end of the DNA sequence shown by SEQ ID NO: 1 (i.e. SEQ ID NO: 3; corresponding to the nucleotides of from No. 301 to No. 1400 in SEQ ID NO: 1);
(F) The DNA fragment of any one of (A) to (E) wherein said portion of the DNA sequence is a sequence comprising at least 1258 bp from the 3'-end of the DNA sequence shown by SEQ ID NO: 1 (i.e. SEQ ID NO: 2; corresponding to the nucleotides of from No. 143 to No. 1400 in SEQ ID NO: 1);
(G) A recombinant DNA fragment comprising said DNA fragment of any one of (A) to (F) and a gene coding for a heterologous protein positioned downstream of said DNA sequence;
(H) An expression plasmid comprising said recombinant DNA fragment of (G);
(I) A host cell transformed with said recombinant DNA fragment of (G) or with said expression plasmid of (H);
(J) The host cell of (I) wherein said host cell is yeast; and
(K) A method, for preparing a heterologous protein which comprises culturing said host cell of (I) or (J) and recovering a heterologous protein from the culture.

### (Effects of the Invention)

The DNA fragment according to the present invention comprises a sequence that exhibits a potent promoter activity when used in the yeast. Thus, by introducing a plasmid comprising the DNA fragment according to the present invention and a heterologous gene bound thereto into a host cell, inter alia a yeast cell, said heterologous gene may be expressed at a high expression level.

### BRIEF DESCRIPTION OF DRAWINGS

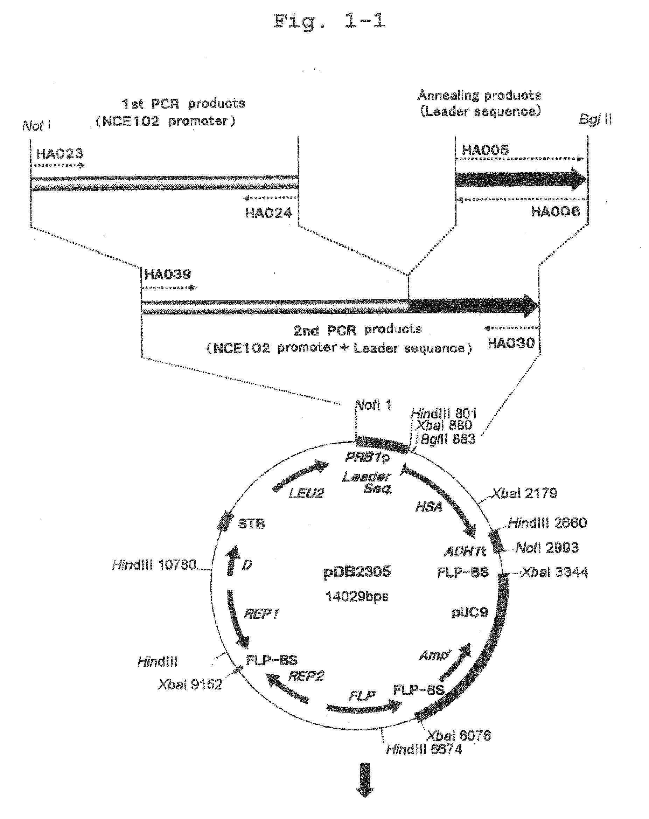

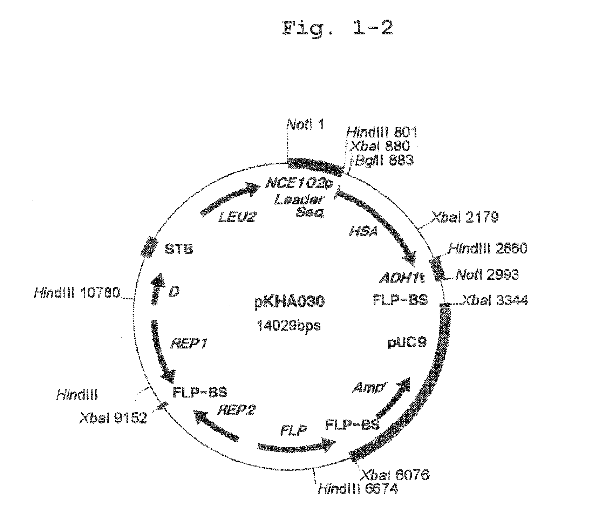

Fig. 1-1 shows construction of the plasmid pKHA030.
Fig. 1-2 shows construction of the plasmid pKHA030 (continued).
Fig. 2 shows various fragments from the promoter NCE102.
Fig. 3 shows results of rocket immunoelectrophoresis with the promoter NCE102 and other promoters for an expression level of rHSA.
Fig. 4 shows a ratio of HSA-mRNA level for the promoter NCE102 and other promoters.
Fig. 5 shows a ratio of HSA-mRNA level for various fragments from the promoter NCE102.

### BEST MODE FOR CA-RRYING OUT THE INVENTION

The DNA fragment with a promoter activity according to the present invention has a whole of the DNA sequence shown by SEQ ID NO: 1 or a portion of said DNA sequence inclusive of its 3'-end. The DNA fragment is positioned upstream of an open reading frame of the NCE102 gene in the yeast and comprises a promoter region.

In addition to the sequence shown by SEQ ID NO: 1, the DNA fragment with a promoter activity according to the present invention is a sequence comprising at least 650 bp from the 3'-end of the DNA sequence shown by SEQ ID NO: 1 (i.e. SEQ ID NO: 6). More preferably, the DNA fragment with a promoter activity according to the present invention is a sequence comprising at least 820 bp from the 3'-end of the DNA sequence shown by SEQ ID NO: 1 (i.e. SEQ ID NO: 5) .

The DNA fragment with a promoter activity according to the present invention further encompasses a DNA sequence which is hybridizable to a sequence complementary to a whole of the DNA sequence shown by SEQ ID NO: 1 or to a portion of said DNA sequence inclusive of its 3'-end and maintains the transcription promoter activity of said whole or portion of the DNA sequence shown by SEQ ID NO: 1. Such a DNA sequence should be construed to encompass a whole of the DNA sequence shown by SEQ ID NO: 1 or a portion of said DNA sequence inclusive of its 3'-end wherein any of the nucleotides therein is subject to deletion, substitution or addition or a combination thereof but the promoter activity is still maintained.

As used herein, "a DNA sequence which is hybridizable" refers to a DNA sequence which is hybridizable under stringent conditions.

As used herein, the term "promoter" refers to any DNA sequence which, when bound to a structural gene in a host cell, may promote transcription, translation, or stability of mRNA for said structural gene as compared to those in the absence of said promoter. Besides, as used herein, the term "a heterologous gene" refers to any gene that does not co-exist functionally with a specific promoter in nature irrespective of whether said gene is derived from a specific organism. Furthermore, as used herein, the terms "a DNA", "a gene" and "a genetic DNA" are used substantially synonymously and interchangeable from each other.

The DNA fragment comprising a yeast promoter according to the present invention may be fully synthesized by the technique of nucleic acid synthesis routinely used in the art.

Introduction of a heterologous gene bound downstream of the DNA fragment comprising a promoter according to the present invention (hereinafter also referred to as "a promoter-bound heterologous gene") into a yeast cell may provide a yeast cell wherein the expression of the heterologous gene is regulated by the promoter according to the present invention.

Introduction of a promoter-bound heterologous gene into a yeast cell may be performed e.g. as taught by Hinnen et al., Proc. Natl. Acad. Sci. USA, 75, 1929-1933, 1978. Specifically, a promoter-bound heterologous gene may be incorporated into a vector which is then introduced into a yeast cell or the gene may directly be introduced into a yeast cell.

In accordance with the present invention, an expression plasmid is provided wherein a DNA, which comprises a DNA fragment comprising a promoter-containing DNA fragment and a. heterologous gene positioned downstream of said promoter, is bound to a vector.

A vector to be used for construction of an expression plasmid includes, for instance, a vector based on a 2 µm DNA of yeast as described by Hinnen et al. as well as vectors commonly used for yeast such as "YRp" vector, a multiple copy vector for yeast wherein an ARS sequence of the yeast chromosome is a replication origin; "YEp" vector, a multiple copy vector for yeast having a replication origin of a 2 µm DNA of yeast; "YCp" vector, a single copy vector having an ARS sequence of the yeast chromosome as a replication origin and a centromeric DNA of the yeast chromosome; and "YIp" vector, a vector for incorporating into the yeast chromosome without a replication origin of yeast .

A heterologous gene to be bound includes, for instance, but is not limited to, a human serum albumin (hereinafter also referred to as "rHSA") gene. A heterologous gene to be bound may be used not only for the expression of a gene product encoded by said heterologous gene but also for the control of expression through an antisense RNA.

An expression vector may further comprise a marker gene for the selection of a transformant such as e.g. LEU2 gene, or a terminator complied with a host cell transformed such as e.g. a terminator of ADH1 gene.

Transformation of a host cell yeast with an expression plasmid may be performed by, but is not limited to, e.g. electroporation.

A host cell yeast to be transformed with an expression plasmid includes, for instance, but is not limited to, S . cerevisiae.

The transformant according to the present invention may be cultured to render a heterologous protein be expressed to thereby produce said heterologous protein. A heterologous protein to be expressed includes, for instance, but is not limited to, a substance useful in the medical field such as a human serum albumin or an immunoglobulin.

The present invention is explained in more detail by means of the following Examples but should not be construed to be limited thereto.

### Example 1: Cloning of promoter and construction of rHSA expression plasmid

PCR (1st PCR) was performed with the genomic DNA of S. cerevisiae S288C strain as a template, synthetic DNAs HA023: ATAGCGGCCGCAGAATGCAATGCGGCTTTTGTTTG (SEQ ID NO:11): and HA024: TATGGGATTATATTGTTATTAGGTATGCTTTGAGA (SEQ ID NO: 12) as a primer, and Pwo DNA polymerase (Roche) under conditions that 30 cycles were repeated with each cycle consisting of three steps of 94°C for 15 seconds, 47°C for 30 seconds and 72°C for 45 seconds, followed by incubation at 72°C for 5 minutes. This 1st PCR produced the 1st PCR product which was electrophoresed on agarose gel at the position corresponding to a size of about 0.83 kbp.

Synthetic DNAs HA005:
ATGAAGTGGGTTTTCATCGTCTCCATTTTGTTCTTGTTCTCCTCTGCTTACTCTA (SEQ ID NO:13) and HA006:
GATCTAGAGTAAGCAGAGGAGAACAAGAACAAAATGGAGACGATGAAAACCCACTTCAT (SEQ ID NO:14) were mixed and the mixture was incubated at 96°C for 10 minutes and then cooled to room temperature for annealing.

The thus annealed synthetic DNA linkers HA005/HA006 and the 1st PCR product were ligated at their blunt ends. PCR (2nd PCR) was then performed with the ligation solution as a template, and synthetic DNAs HA030: ATCCAAAGATCTAGAGTAAGCAGAGGAGAACAAGAAC (SEQ ID NO:15) and HA039:AAGGAAAAAAGCGGCCGCAGAATGCAATGCGGCTTTTGTTTG (SEQ ID NO: 16) as a primer under conditions that 30 cycles were repeated with each cycle consisting of three steps of 94°C for 15 seconds, 53°C for 30 seconds and 72°C for 45 seconds, followed by incubation at 72°C for 2 minutes. This 2nd PCR produced the 2nd PCR product which was electrophoresed on agarose gel at the position corresponding to a size of about 0.83 kbp.

The purified 2nd PCR product was digested with restriction enzymes NotI and BglII and electrophoresed on agarose gel to cleave the digestion product at the position corresponding to a size of about 0.90 kbp.

This digestion product was replaced for a NotI-BglII region comprising the PRB1 promoter of the rHSA expression plasmid pDB2305, which was derived from the rHSA expression plasmid pDB2244 (WO00/44772), to produce a novel rHSA expression plasmid pKHA030.

The cloning of the promoter according to the present invention and the construction of the rHSA expression plasmid as described above are illustrated in Fig. 1.

### Example 2: Determination of nucleotide sequence of promoter

A nucleotide sequence of the region as cloned in Example 1 was determined, following PCR with pKHA030 as a template and using CEQ DTCS-Quick Start Kit (Beckman Coulter K. K.), by the method of Sanger (Proc. Natl. Acad. Sci. USA, 74, 5463-5467, 1977).

### Example 3: Modification of promoter region

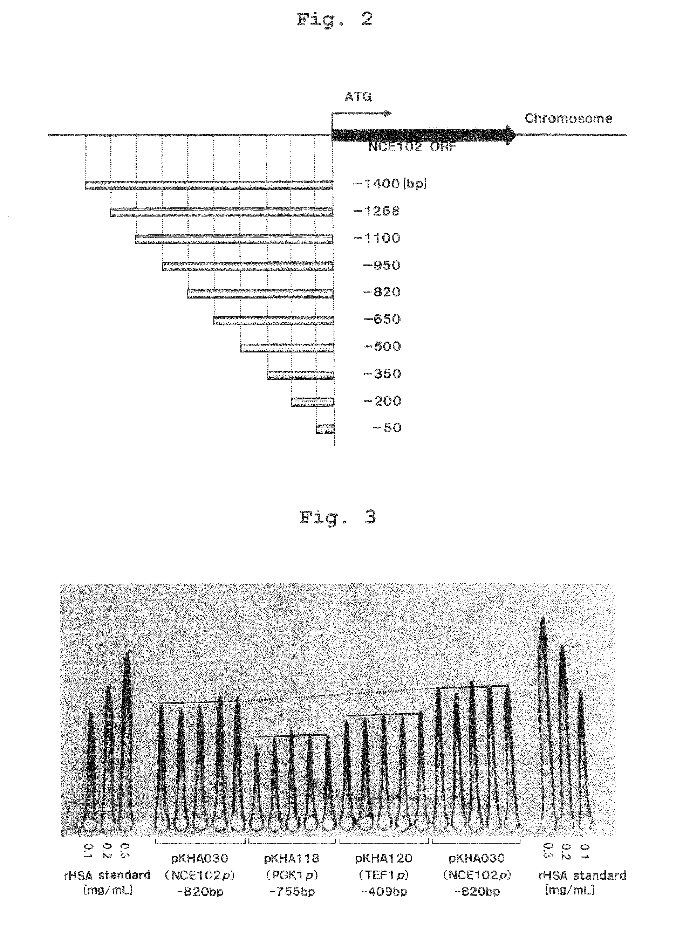

In addition to the promoter region (820 bp) in the genomic DNA of the yeast S. cerevisiae S288C strain in accordance with the present invention, the present inventors further considered additional 580 bp upstream of said promoter region and prepared this 1400 bp region and its various fragments with various degrees of deletion at the 5' terminal. For definition of these fragments, the translation initiation point of the promoter of the present invention ("A" of AUG codon) was set as zero and the promoter region upstream thereof (820 bp) was termed "-820 bp" (SEQ ID NO: 5; corresponding to the nucleotides of from No. 581 to No. 1400 in SEQ ID NO: 1). Each of the following DNA fragments were prepared as described in Examples 1 and 2 (Fig. 2) and the corresponding rHSA expression plasmids were constructed (Table 1): "-1400 bp" (the DNA sequence of SEQ ID NO: 1; corresponding to the nucleotides of from No. 1 to No. 1400 in SEQ ID NO: 1); "-1258 bp" (SEQ ID NO: 2; corresponding to the nucleotides of from No. 143 to No. 1400 in SEQ ID NO: 1); "-1100 bp" (SEQ ID NO: 3; corresponding to the nucleotides of from No. 301 to No. 1400 in SEQ ID NO: 1); "-950 bp" (SEQ ID NO: 4; corresponding to the nucleotides of from No. 451 to No. 1400 in SEQ ID NO: 1); "-650 bp" (SEQ ID NO: 6; corresponding to the nucleotides of from No. 751 to No. 1400 in SEQ ID NO: 1); "-500 bp" (SEQ ID NO: 7; corresponding to the nucleotides of from No. 901 to No. 1400 in SEQ ID NO: 1); "-350 bp" (SEQ ID NO: 8; corresponding to the nucleotides of from No. 1051 to No. 1400 in SEQ ID NO: 1) ; "-200 bp" (SEQ ID NO: 9; corresponding to the nucleotides of from No. 1201 to No. 1400 in SEQ ID NO: 1); "-50 bp" (SEQ ID NO: 10; corresponding to the nucleotides of from No. 1351 to No. 1400 in SEQ ID NO: 1); and "-0 bp".

**Table 1**

| rHSA expression plasmids | NCE102 promoter regions [bp] |
|---|---|
| pKHA063 | -1400 |
| pKHA065 | -1258 |
| pKHA067 | -1100 |
| pKHA069 | -950 |
| pKHA030 | -820 |
| pKHA071 | -650 |
| pKHA073 | -500 |
| pKHA075 | -350 |
| pKHA077 | -200 |
| pKHA079 | -50 |
| pKHA099 | 0 |

### Example 4: Screening of yeast transformant

The plasmid pKHA030 and each of the rHSA expression plasmids as constructed in Example 3 were introduced, into S. cerevisiae strain (cir⁰ a, leu2-3, leu2-112, can1, pra1, yap3, hsp150) as described by Hinnen et al. The resulting transformants were screened on a buffered minimum agar medium defective in leucine [0.15% (w/v) yeast nitrogen base free from amino acids and ammonium sulfate, 0.5% (w/v) ammonium sulfate, 36 mM citric acid, 126 mM sodium dihydrogenphosphate, pH 6.5, 2% (w/v) sucrose, and 1% (w/v) Bacto agar].

### Example 5: Estimation of expression level of secreted rHSA

The obtained transformant was cultured in a. 50 mL flask containing 10 mL of a buffered minimum liquid medium [0.15% (w/v) yeast nitrogen base free from amino acids and ammonium sulfate, 0.5% (w/v) ammonium sulfate, 36 mM citric acid, 126 mM sodium dihydrogenphosphate, pH 6.5, and 2% (w/v) sucrose] at 30°C, 200 rpm for 96 hours. The expression level of rHSA was estimated by rocket immunoelectrophoresis. Initially, the culture (N=5) were centrifuged and the respective supernatant were recovered. Each 4 µL of these supernatant were added to 1.0% (w/v) agarose gel containing anti-human albumin antibody (Sigma) and electrophoresed. The gel after electrophoresis was stained with Coomassie Brilliant Blue (R-250) and rHSA levels in each of the culture supernatant were compared by observing the height of the obtained rocket-like peaks.

As shown in Fig. 3, an rHSA level expressed from the yeast where the plasmid pKHA030 containing the NCE102 promoter (-820bp) was introduced was significantly higher than those expressed from the yeast where the plasmid containing the PGK1 promoter or TEF1 promoter was introduced.

### Example 6: Estimation of transcription level of HSA-mRNA

The transformant obtained in Example 4 was cultured in a 50 mL flask containing 10 mL of a buffered minimum agar medium [0.15% (w/v) yeast nitrogen base free from amino acids and ammonium sulfate, 0.5% (w/v) ammonium sulfate, 36 mM citric acid, 126 mM sodium dihydrogenphosphate, pH 6.5, and 2% (w/v) sucrose] at 30°C, 200 rpm and the yeast cells were recovered after 72 hours. Whole RNAs were extracted from the recovered cells with RNeasy Mini Kit (QIAGEN) and treated simultaneously with DNase.

A transcription activity of the promoters was compared by LightCycler System (Roche) with QuantiTect SYBR Green RT-PCR Kit (QIAGEN) using the extracted whole RNAs as a template and synthetic DNAs HA045:
GTCCGAAGTCGCTCACAGATTCAA (SEQ ID NO: 17) and HA046:
GCAGATTCGTCAGCAACACAAGTC (SEQ ID NO: 18) for detection of the HSA gene or synthetic DNAs HA062:
GCGTGTCTTCATCAGAGTTGACTTC (SEQ ID NO: 19) and HA063:
CCAAGTGAGAAGCCAAGACAACGTA (SEQ ID NO: 20) for detection of the PGK1 gene. The mixture was initially incubated at 50°C for 20 minutes for reverse transcription and then thermal denaturation was performed at 96°C for 15 minutes. Then, 40 cycles were repeated with each cycle consisting of three steps of 94°C for 15 seconds, 53°C for 20 seconds and 72°C for 10 seconds to determine increased levels of the HSA gene fragment for HA045/HA046 and of the PGK1 gene fragment for HA062/HA063 with supposition that an absolute value of the HSA-cDNA level and the PGK1-cDNA level in each sample was equal to their respective mRNA levels. A transcription activity of the promoters was numerically expressed by a ratio of HSA-mRNA level/PGK1-mRNA level (hereinafter referred to as "a ratio of HSA-mRNA level").

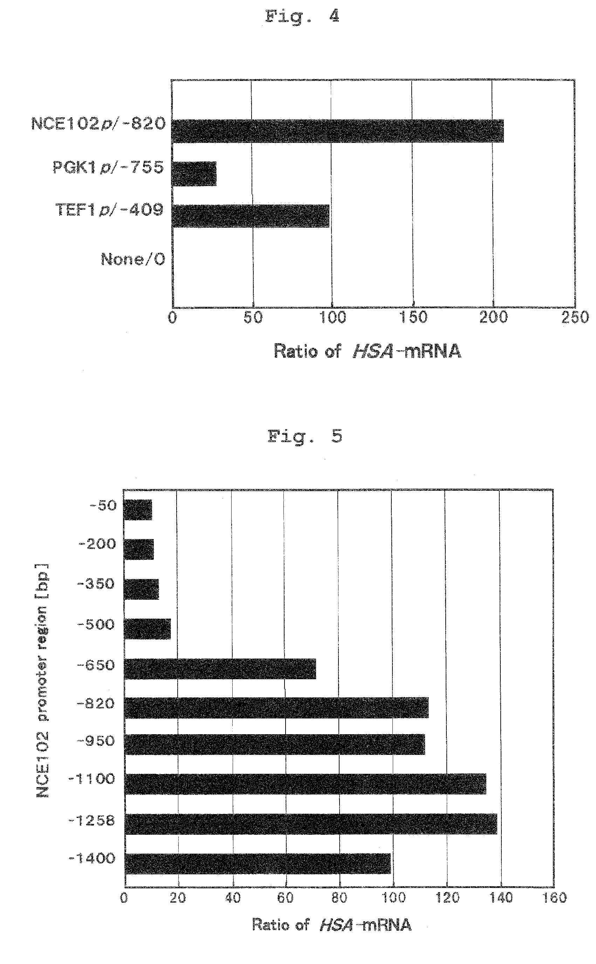

A ratio of HSA-mRNA level in the yeast where the plasmid pKHA030 containing the NCE102 promoter (-820bp) was introduced was apparently higher than those in the yeast where the plasmid containing the PGK1 or TEF1 promoter was introduced (Fig. 4).

When various fragments of the NCE102 promoter were prepared, a ratio of HSA-mRNA level was as shown in Fig. 5 wherein the significantly higher level was observed for the promoter fragment longer than -650 bp (SEQ ID NO: 6; corresponding to the nucleotides of from No. 751 to No. 1400 in SEQ ID NO: 1)

## Claims

1. A DNA fragment possessing a transcription promoter activity of yeast, said DNA fragment having a whole of the DNA sequence shown by SEQ ID NO: 1 or a portion of said DNA sequence inclusive of its 3'-end, or having a DNA sequence which is hybridizable to a sequence complementary to a whole of the DNA sequence shown by SEQ ID NO: 1 or to a portion of said DNA sequence inclusive of its 3'-end and maintains the transcription promoter activity of said whole or portion of the DNA sequence shown by SEQ ID NO: 1.

2. The DNA fragment of claim 1 wherein said portion of the DNA sequence is a sequence comprising at least 650 bp from the 3'-end of the DNA sequence shown by SEQ ID NO: 1 (i.e. SEQ ID NO: 6).

3. The DNA fragment of claim 1 or 2 wherein said portion of the DNA sequence is a sequence comprising at least 820 bp from the 3'-end of the DNA sequence shown by SEQ ID NO: 1 (i.e. SEQ ID NO: 5).

4. The DNA fragment of any one of claims 1 to 3 wherein said portion of the DNA sequence is a sequence comprising at least 950 bp from the 3'-end of the DNA sequence shown by SEQ ID NO: 1 (i.e. SEQ ID NO: 4) .

5. The DNA fragment of any one of claims 1 to 4 wherein said portion of the DNA sequence is a sequence comprising at least 1100 bp from the 3'-end of the DNA sequence shown by SEQ ID NO: 1 (i.e. SEQ ID NO: 3).

6. The DNA fragment of any one of claims 1 to 5 wherein said portion of the DNA sequence is a sequence comprising at least 1258 bp from the 3'-end of the DNA sequence shown by SEQ ID NO: 1 (i.e. SEQ ID NO: 2).

7. A recombinant DNA fragment comprising said DNA fragment of any one of claims 1 to 6 and a gene coding for a heterologous protein positioned downstream of said DNA sequence.

8. An expression plasmid comprising said recombinant DNA fragment of claim 7.

9. A host cell transformed with said recombinant DNA fragment of claim 7 or with said expression plasmid of claim 8.

10. The host cell of claim 9 wherein said host cell is yeast.

11. A method for preparing a heterologous protein which comprises culturing said host cell of claim 9 or 10 and recovering the heterologous protein from the culture.
